Europäisches Patentamt

European Patent Office

Office européen des brevets

(19)

(11) Veröffentlichungsnummer: **0 152 106 B1**

(12) **EUROPÄISCHE PATENTSCHRIFT**

(45) Veröffentlichungstag der Patentschrift: 25.09.91

(51) Int. Cl.⁵: **A61K 31/355**, A61K 9/06

(21) Anmeldenummer: 85101554.5

(22) Anmeldetag: 13.02.85

(54) **Rheumamittel.**

(30) Priorität: 15.02.84 DE 3405240
27.02.84 DE 3407024
07.03.84 DE 3408260
24.04.84 DE 3415250
02.05.84 DE 3416162
07.08.84 DE 3429019
07.09.84 DE 3432881
07.09.84 DE 3432882

(43) Veröffentlichungstag der Anmeldung:
21.08.85 Patentblatt 85/34

(45) Bekanntmachung des Hinweises auf die
Patenterteilung:
25.09.91 Patentblatt 91/39

(84) Benannte Vertragsstaaten:
AT BE CH DE FR GB IT LI LU NL SE

(56) Entgegenhaltungen:
EP-A- 0 151 987        EP-A- 0 151 989
DE-A- 1 617 418        DE-A- 2 425 222
FR-A- 2 300 554        GB-A- 1 135 709
US-A- 4 169 143

ROTE LISTE, 1983, EDITIO CANTOR,
AULENDORF/WÜRTT., DE

DICTIONNAIRE VIDAL, 1982, ed. 58, OVP, Paris, FR

B. HELWIG: "Moderne Arzneimittel", 5. Auflage 1980, Wissenschaftliche Verlagsgesellschaft, Stuttgart, DE

idem

SEIFEN, ÖLE, FETTE, WACHSE, Band 96, Nr.
4, 1970, Augsburg, DE

WO 84/01899

(73) Patentinhaber: Ismail, Roshdy, Dr.
Siebengebirgs-Apotheke Siebengebirgsallee
2
W-5000 Köln 41 (Klettenberg)(DE)

(72) Erfinder: Ismail, Roshdy, Dr.
Siebengebirgs-Apotheke Siebengebirgsallee
2
W-5000 Köln 41 (Klettenberg)(DE)

(74) Vertreter: Werner, Hans-Karsten, Dr. et al
Deichmannhaus am Hauptbahnhof
W-5000 Köln 1(DE)

EP 0 152 106 B1

## Beschreibung

Die vorliegende Erfindung betrifft eine neue Verwendung von Vitamin E.

Vitamin E ist bekannt als Antioxidans und Schutzvitamin für Phospholipide der Zellmembran. Es hält die Permeabilität und Stabilität der Zellmembran aufrecht; Lucy, Ann. N.Y. Academy of Science 203 1972, S. 4. Es ist weiterhin bekannt, daß Vitamin E eine membranabdichtende Wirkung besitzt; F. Mittelbach und G. Bodechtel, Münchner Medizinische Wochenschrift 110 (1968) 36: 1988-1993. Bei Erythrocyten, den einfachsten Zellen des menschlichen Körpers wurde festgestellt, daß Vitamin E eine Schutzwirkung für die Zellmembran erbringt. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685-704 (1968) und Phelps DL Pediatrics 63 (6) S. 933-935 (1979). Aus diesen Literaturstellen geht hervor, daß bei Gabe von 200 bis 800 mg Vitamin E oral für einen Zeitraum von 1 bis 4 Tagen, die Hämolyse der Erythrocyten signifikant verbessert wird im Vergleich zu Patienten mit Vitamin-E-Mangel.

Weiterhin ist bekannt, daß 750 mg Vitamin E täglich in einem Zeitraum von 3 bis 6 Monaten erfolgreich bei Thalassamie-Patienten eingesetzt wurde, wobei eine Normalisierung der Hämolyse der Erythrocyten beobachtet wurde; vgl. Kahane I. ISR. J. med. 12 (1), S. 11-15 (1976).

Erfolgreich eingesetzt wurde Vitamin E weiterhin bei Patienten mit akuter Hepatitis und alkoholischer Hepatitis, die einen Mangel an Vitamin E im Serum haben;

vgl. Yoshiakawa T. Takemura S. Kato H. et al. Japan J. Gastrovent, 74/7, S. 732-739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipidmetabolismus im Knochenmark; vgl. Takoshi Itaga, Central Clinical Laboratory Nagasaki University of Medicine, Japan. In der WO 84/01899 wird die Verwendung eines Vitamins, bevorzugt der B-Reihe, oder einer Kombination von Vitaminen zur Behandlung und Prophylaxe rheumatischer Erkrankungen offenbart. Neben mehreren anderen Vitaminen wird auch Vitamin E in Mengen von etwa 10 mg pro Darreichungseinheit vorgesehen.

Es wurde überraschenderweise gefunden, daß Vitamin-E-Kombinationen mit gefäßerweiternden und/oder durchblutungsfördernden Mitteln zur Behandlung von Rheumaerkrankungen geeignet sind. Dieser neue Indikationsbereich war aufgrund des bisherigen Wissenstandes nicht vorherzusehen und eröffnet ein neues breites Anwendungsfeld für Vitamin E.

Die vorliegende Erfindung betrifft die Verwendung von Vitamin E in Kombination mit gefäßerweiternden und/oder durchblutungsfördernden Mitteln, und gegebenenfalls einem oder mehreren Stoffen aus der Gruppe Lecithin, Vitamin A, Vitamine der B-Reihe, Analgetika, Emulgatoren, Lösungsvermittler, Antikoagulantien und übliche Träger- und Hilfsstoffe zur Herstellung eines Arzneimittels gegen Rheumaerkrankungen.

Unter rheumatischen Erkrankungen versteht man Schmerzen und meist auch Bewegungseinschränkungen. Die Ursachen sind sehr verschiedenartig, wie z.B. Zephalgie, Brachialgie, Lumbalgie, Kardialgie, Nephralgie, Myalgie auch Neuralgien, Schmerzen im Ausbreitungsgebiet peripherer Nerven.

Es wurde überraschenderweise gefunden, daß die Wirkung von Vitamin E in Gegenwart von gefäßerweiternden und/oder durchblutungsfördernden Mitteln erheblich gesteigert wird, und dadurch die Behandlungszeit verkürzt wird. Die Symptome gehen schneller zurück. Die Verwendung von Vitamin-E-haltigen Kombinationspräparaten muß jedoch längere Zeit, ca. 6 Monate oder länger, erfolgen.

Im Falle topisch anwendbarer Mittel bzw. deren Verwendung wird das Eindringen von Vitamin E durch die Haut überraschenderweise durch die Gegenwart von durchblutungsfördernden Mitteln, wie Heparin Natrium, Extr. Hippocastani etc., besonders erhöht und demzufolge in seiner Wirkung erheblich gesteigert. Bei der Verwendung von Heparin Natrium wird die hohe Dosierung von 30.000 bis 150.000 I.E. bevorzugt.

Es wurde gefunden, daß bei diesen Wirkstoffen in Kombination mit ausreichend dosiertem Vitamin E die Behandlungsdauer wesentlich verkürzt werden kann. Die Krankheitsymptome gehen rascher zurück, so daß nach einiger Zeit auch die Dosierung reduziert werden kann.

Die Ergebnisse waren nicht vorhersehbar und ermöglichen eine Therapie, bei der ein Teil des chemischen Wirkstoffes durch einen Naturstoff ersetzt wird, der sich obendrein praktisch in jeder Körperzelle befindet.

Die vorliegende Erfindung beschreibt in einer bevorzugten Ausführungsform die Verwendung von Vitamin E zusammen mit gefäßerweiternden Mitteln für die Herstellung von Rheumamitteln zur äußerlichen Anwendung, wie z.B. Cremes, Gele und Salben oder Lotionen.

Mittel, die bei Verwendung von Vitamin E für äußerlich anwendbare Arzneimittel die Wirkung von Vitamin E erheblich steigern und dadurch erfindungsgemäß verwendet werden können, sind durchblutungsfördernde Mittel wie Heparin-Natrium, Extract. Hippocastani, $\beta$-Hydroxyäthyl-rutosid, Extractum Arnicae, Extract. Capsicae, Ol. Juniperi, Ol. pinipumilionis, Ol. Eucalypti, Ol. Rosmarinae, Tinct. Camphorae oder

2

Campher, Extract. Calendulae, Trimethylolrutosid, Benzylnicotinat, Inositolnicotinat, Xantinolnicotinat, β-Hydroxyethylsalicylat, Pentoxyfyllin, Bamethansulfat, Buflomedil, Bencyclanfumarat, Bencyclanhydrogenfumarat, Flunarizin, β-Pyridylcarbinol, Nicotinsäure, Nicotinsäureester sowie Salicylsäure bzw. deren Ester, Dihydroergotoxin-methan-sulfonat, Dihydroergocornin-methan-sulfonat, Dihydroergocristin-methan-sulfonat und Ginkoflavonglykoside. Es wurde nun gefunden, daß die Verwendung dieser Mittel in Kombination mit ausreichend dosiertem Vitamin E die Symptome bei zahlreichen Patienten rascher zurückgehen und nach mehreren Monaten die Menge dieser durchblutungsfördernden Mittel verringert werden kann.

Außer den vorgenannten gefäßerweiternden bzw. durchblutungsfördernden Mitteln können ebenfalls andere gleichartige Produkte wie Cinnarizin, Vincamin etc. verwendet werden.

Bei der Rheumasalbe oder Gel bzw. Creme wird Vitamin E in Form des freien Tocopherols, wie z.B. D,L-alpha-Tocopherol und D-alpha-Tocopherol, verwendet.

Als übliche Salben- oder Cremegrundlagen können Eucerin cum aqua, Ungt. Cordes, Ungt. Emulsificans, sowie andere nicht wasserlösliche Salbengrundlagen bzw. deren Gemische verwendet werden. Geeignete Salbengrundlagen sind beispielsweise Wollwachs, Vaseline DAB 8, Paraffin dünnflüssig sowie Gemische derselben. Sie können auch Emulgatoren enthalten wie Cetylstearylalkohol etc.. Auch geeignet als Salbengrundlagen sind Unguentum alcoholum lanae aquosum mit ca. 5 bis 10% Cetiol (Ölsäureoleylester), sowie unguentum lanette, 24 T Cetylstearylalkohol, 16 T Cetiol DAB 8, 60 T aqua conservata.

Bei dieser Kombination zieht Vitamin E sofort in die Haut ein. Zu dieser Kombination von Vitamin E mit gefäßerweiternden Mitteln können selbstverständlich weitere Vitamine, z.B. Vitamin $B_1$, $B_2$ und $B_6$ und verträgliche Schmerzmittel sowie Lokalanaesthetika zugesetzt werden. Auch Lokalanaesthetika sind gefäßerweiternd. Sie können zu den Salben als Oberflächenanaesthetikum, wie Anaesthesin (Ethaform) sowie Tetracain(Pantocain), zugegeben werden, oder in die Kapseln, wie Procain bzw. Procainhydrochlorid, etc..

Die genannten Salben können beispielsweise als Grundlage vorzugsweise enthalten:

70 bis 30 Gew.-% Wasser, noch mehr vorzugsweise

60 bis 40 Gew.-%

30 bis 5 Gew.-% Cetiol[R] (oleyl oleat), noch mehr vorzugsweise 25 bis 7 Gew.-%

30 bis 2 Gew.-% Cetyl-Stearylalkohol oder andere aliphatische Alkohole, noch mehr vorzugsweise 25 bis 2 Gew.-%.

Man kann anstelle von Cetyl-Stearylalkohol auch ganz oder teilweise andere emulgierende Alkohole, z.B. aliphatische Alkohole oder Wollwachsalkohol bzw. Diole, Stearinol, Monoglyceride mit aliphatischen Säuren verestert oder ähnliche Stoffe verwenden. Man kann z.B. auch Paraffin oder Vaseline zusetzen, um die Salbe streichfähig zu machen. Auch Cetiol[R] (oleyl oleat) kann mit anderen Emulgatoren, z.B. Tween[R] 20 oder Tween[R] 80 etc., ganz oder teilweise ersetzt werden. Es wurde jedoch gefunden, daß eine besonders bevorzugte Kombination als Grundlage für Vitamin-E-haltige Salben bzw. Cremes folgende ist:

30 bis 20 Gew.-% Cetyl-Stearylalkohol,

20 bis 10 Gew.-% Cetiol[R] (oleyl oleat)

60 bis 40 Gew.-% Wasser (aqua conservata).

Diese Vitamin-E-haltige Salbe zieht sofort in die Haut ein.

Es ist bekannt, daß wasserhaltige Salbengrundlagen, wie Unguentum emulsificans aquosum und Unguentum alcoholum lanae aquosum geeignet sind zur Verarbeitung von wasserlöslichen Wirkstoffen.

Hier überrascht, daß wasserhaltige Salbengrundlagen, die fast über 50% wasserhaltig sind, sehr gut geeignet sind zur Verarbeitung fettlöslicher Wirkstoffe wie Vitamin E.

Erfindungsgemäß bevorzugt wird Vitamin E in Kombination mit gefäßerweiternden Mitteln verwendet, wobei die Menge an Vitamin E in Form von freiem alpha-Tocopherol im Bereich zwischen 5 und 20 Gew.-%, vorzugsweise im Bereich von 5 bis 10 Gew.-%, besonders bevorzugt im Bereich von 8 Gew.-%, bezogen auf die Gesamtmenge aller Komponenten, liegt.

Bei oralen Rheumamitteln ist für die Wirksamkeit von Vitamin E bei der Kombination mit gefäßerweiternden und/oder durchblutungsfördernden Mitteln vor allem eine ausreichende Dosierung entscheidend, die mindestens 150 mg betragen sollte. Niedrigere Dosierungen an Vitamin E sind nutzlos, da große Teile durch die Magensäure zerstört werden und dadurch ihre Wirksamkeit verlieren; vgl. Arthur Vogelsang in Angiology 21, S. 275-279 (1970).

Sofern in der Vergangenheit hin und wieder geringe Mengen Vitamin E, nämlich bis zu 40 mg in Kombinationspräparaten zum Einsatz gekommen sind, waren sie mit Sicherheit wegen der zu niedrigen Dosierung völlig wirkungslos. Zur Behandlung von Rheumaerkrankungen sollte die Dosierung dabei im Bereich von 150 bis 600 mg liegen. Vorzugsweise werden Darreichungsformen, die 150 bis 500 mg enthalten, eingesetzt. Typische Kombinationspräparate enthalten 200 bis 400 mg Vitamin E. Insbesondere bei den Kombinationen mit Nicotinsäure und deren Derivaten werden hohe Dosierungen an Vitamin E,

3

zwischen 300 und 500 mg pro Darreichungsform, benötigt.

Als Vitamin E bei der oralen Darreichungsform kann sowohl der Ester aus natürlicher oder synthetischer Herkunft als auch das freie Tocopherol verwendet werden.

Die erfindungsgemäßen Mittel enthalten außer den Wirkstoffen und Vitamin E übliche Träger- und Hilfsstoffe. Da Vitamin E bei üblichen Temperaturen flüssig ist, bietet sich hierfür als Applikationsform insbesondere die Weichgelatinekapsel an. Die übrigen Wirkstoffe werden in Vitamin E sowie gewünschtenfalls in einem dünnflüssigen Neutralöl und einem Lösungsvermittler in an sich bekannter Weise in Weichgelatinekapseln eingebracht, auch hier können geeignete Emulgatoren, wie z.B. Tween(R), eingesetzt werden. Hierbei können insbesondere die Standardrezepturen der Firma Scherer, Eberbach zur Anwendung kommen. Die Verwendung dieser Kombinationen in Form von Tropfen, z.B. als alkoholische Lösung, kann ebenfalls geeignet sein.

Überraschenderweise bringt die Verwendung der Rheumamittel gemäß der Erfindung besondere Vorteile, wenn Vitamin A zugesetzt wird. Insbesondere wird die Behandlungsdauer verkürzt. Demzufolge beschreibt die vorliegende Erfindung auch die Verwendung von Kombinationen, die Vitamin A und E und durchblutungsfördernde Mittel enthalten. Vitamin A und E neigen insbesondere in Gegenwart von anderen Wirkstoffen im wässrigen Medium sehr stark zu Klumpenbildung. Dabei besteht die Gefahr, daß die fettlöslichen wertvollen Stoffe nicht absorbiert werden.

Überraschend wurde festgestellt, daß geringe Mengen Emulgator, ca 1%, ausreichen, um die Klumpenbildung zu verhindern. Die Wirkstoffe werden leichter im wässrigen Medium dispergiert bzw. suspendiert. Dies hat den Vorteil, daß die Absorption durch den Darm erleichtert wird. Eine größere Menge Emulgator ist nicht notwendig, da meistens 1 bis 7% ausreichend sind, um die Klumpenbildung zu verhindern. Man kann auch Mengen bis zu 10% oder mehr verwenden. Dabei besteht aber die Gefahr, daß man zu viele Hilfsstoffe zugibt. Die Folge können Nebenwirkungen sein, insbesondere wenn das Medikament längere Zeit eingenommen wird.

Es können die üblichen Emulgatoren, die in den medizinischen Präparaten verwendet werden, wie Tween(R) 20, Chremophore(R), aliphatische Alkohole, partialveresterte Triglyceride etc. Für diese Erfindung werden jedoch Tween(R) 80 und Cetiol(R) bevorzugt. Hierbei wurde beobachtet, daß bei Zugabe von ca. 10% Emulgator die Emulgierung nicht wesentlich besser ist als bei Zusatz von 5% Emulgator.

Man kann als Emulgator auch Lecithin in einer Konzentration zwischen 1 und 13% verwenden. Damit wird die Resorption von der Kombination A + E, insbesondere von A begünstigt. Geringe Mengen des Emulgators aus Lecithin sind ausreichend, um die Klumpenbildung der fettlöslichen Vitamine zu verhindern und um eine optimale Resorption zu begünstigen. Zwar ist auch bei Verwendung von großen Mengen Lecithin, bis zu 50%, eine positive Wirkung erkennbar, es ist aber zu empfehlen, ca. 1% herkömmliche Emulgatoren wie Tween(R) 80 beizufügen, da sie die Mischbarkeit von Lecithin mit den beiden Vitaminen begünstigen und eine Klumpenbildung verhindern. Besonders vorteilhaft für die Resorption ist die Verwendung von ca. 1% Tween(R) 80 mit 1 bis 13% Lecithin. Ebenso können die herkömmlichen Emulgatoren, Tween(R) 20, Cetiol(R), Ölsäureoleylester und Cremophore(R) verwendet werden. Als Lecithinpräparat wird das Sojalecithin bevorzugt.

Vitamin E kann in allen seinen alpha-Formen verwendet werden, sowohl als freies Tocopherol als auch als Ester. Dieser Ester kann als Acetat, Succinat oder als anderer Ester verwendet werden. Man kann andere Darreichungsformen zubereiten, wie Tabletten oder Drageés, wenn man Vitamin E in fester Form verwendet. Auch als alkoholische Lösung ist es zu verwenden. Die Menge des Vitamin E soll hoch dosiert sein, d.h. zwischen 150 und 600 mg, vorzugsweise zwischen 200 bis 600 mg, noch mehr vorzugsweise zwischen 300 bis 500 mg pro Darreichungsform.

Vitamin A kann in Form von Vitamin-A-Palmitat, Vitamin-A-Acetat, weiterer Ester des Vitamin A, oder $\beta$-Carotin verwendet werden.

Vitamin A soll so ausgewählt sein, daß die maximale Tagesdosis 50.000 I.E. nicht überschreitet, d.h., wenn zwei Darreichungsformen pro Tag angewandt werden, soll die Dosierung bei maximal 25.000 I.E. pro Darreichungsform liegen.

Es können auch weitere Zusätze, z.B. Vitamine der B-Reihe oder Analgetika zugesetzt werden. Als durchblutungsfördernde Mittel können vorzugsweise folgende Stoffe bzw. deren Derivate verwendet werden:

Extract. Hippocastani, Extract. Arnicae, Salicylsäure oder deren Ester, $\beta$-Hydroxyethylsalicylat, Flunarizin, Nicotinsäure oder deren Ester, Benzylnicotinat, Inositolnicotinat, Xantinolnicotinat, Buflomedil, Cinnarizin, Bencyclanhydrogenfumarat, Bencyclanfumarat, Vincamin, Dihydroergotoxinmethansulfonat, Pentoxyfyllin, $\beta$-Pyridylcarbinol, Bamethansulfat, Ginkoflavonglykoside, $\beta$-Hydroxäthylrutosid und Trimethylolrutosid.

Die durchblutungsfördernden Mittel können ebenfalls in Retard-Form verändert werden.

Insbesondere wurden Erfolge bei der Verwendung von Vitamin E in Kombination mit gefäßerweiternden Mitteln zur Herstellung eines Arzneimittels für die Behandlung von Rheumaerkrankungen in Form rektal anwendbarer Darreichungsformen, bevorzugt Suppositorien, erzielt. Es mußten jedoch bei den Einsatzgebieten bzw. bei den verschiedenen Anwendungen die geeigneten Zusatzstoffe zur unterstützenden Behandlung ausgewählt werden. Die üblichen Hilfsstoffe sowie Trägerstoffe können bei der Herstellung von Suppositorien eingesetzt werden.

Die Kombination mit durchblutungsfördernden Mitteln, z.B. Extract Hippocastani oder β-Hydroxyäthylrutosid bzw. auch Rutosidderivate, die mehrere Hydroxyethyl-Gruppen enthalten, kann verwendet werden. Nicotinsäure bzw. deren Ester, z.B. Nicotinsäurebenzylester oder Nicotinsäure-β-Hydroxyläthylester etc. können Vorteile bringen und die Wirkung von Vitamin E steigern und den Heilungsprozeß verkürzen. Der Vorteil der Verwendung von Suppositorien liegt darin, daß Vitamin E nicht durch die Magensäure zerstört wird. Durch die Verwendung von Emulgatoren, z.B. Ölsäurealkylester wird die Resorption gesteigert.

Bei Rheumapflastern, die eine weitere Applikationsform zur Verwendung von Vitamin E in Kombination mit gefäßerweiternden und/oder durchblutungsfördernden Mitteln gemäß der Erfindung darstellen, wird Vitamin E in Form von D-alpha-Tocopherol oder D,L-alpha-Tocopherol in einer Menge zwischen 0,02 bis 4 g, vorzugsweise 0,1 bis 3 g, in Kombination mit gefäßerweiternden Mitteln, wie Extract Arnicae und durchblutungsfördernden Mitteln, wie Extract Hippocastani oder Extract Capsicae, zugesetzt. Die Kombination mit schmerzlindernden Mitteln, wie Extract Belladonae, wird bevorzugt.

In den nachfolgenden Beispielen wird die erfindungsgemäße Verwendung von Vitamin E mit gefäßerweiternden Mitteln zur Herstellung von Rheumamitteln näher erläutert:

**Beispiel1**
100 g Salbe enthalten:
400 mg Allantoin;
400 mg Dexapanthenol;
5000 mg D-alpha-Tocopherol;
30000 I.E. Heparin Natrium;

**Beispiel2**
100 g Salbe enthalten:
2,5 g O-(β-Hydroxyäthyl)-Rutoside;
6,5 g D-alpha-Tocopherol oder D,L-alpha-Tocopherol;

**Beispiel3**
100 g Salbe enthalten:
400 mg Allantoin;
400 mg Dexapanthenol;
8,8 g D-alpha-Tocopherol oder D,L-alpha-Tocopherol;
30000 I.E. Heparin Natrium

**Beispiel4**
100 g Salbe enthalten:
4,5 g Extract Hippocastani (enthält ca. 800 mg Aescin);
5,0 g D-alpha-Tocopherol;

**Beispiel5**
100 g Gel enthalten:
50000 I.E. Heparin Natrium;
12 g Arnikablüten-Extract ((1:10) Alkohol 60%);
25 g Tinct. Hippocastani e sem. 1:1 entspricht 0,65 g Aescin;
7,5 g D-alpha-Tocopherol;

**Beispiel6**
100 g Gel enthalten:
7,0 g β-Hydroxyethyl-Salicylat;
7,0 g D-alpha-Tocopherol;

**Beispiel7**
Rheumapflaster 15 x 25 cm enthält bzw. ist einseitig präpariert mit:

70 mg Extract Arnicae;
70 mg Extract Capsici;
30 mg Extract Belladonae;
1500 mg D-alpha-Tocopherol-Konzentrat;

**Beispiel8**
100 g Salbe enthalten:
10 g Benzocain (Anaesthesin);
8 g D-alpha-Tocopherol-Konzentrat;
1 g Benzylnicotinat;

**Beispiel9**
100 g Salbe enthalten:
3 g Hydroxyethyl-Salicylat;
1 g Benzylnicotinat;
7 g D-alpha-Tocopherol;

**Beispiel10**
100 g Salbe enthalten:
8 g D-alpha-Tocopherol;
400 mg Allantoin;
400 mg Dexapanthenol;
150000 I.E. Heparin Natrium;

**Beispiel11**
Kapseln enthalten:
250 mg Nicotinsäure;
400 mg D,L-alpha-Tocopherolacetat;
150 mg Sojabohnenöl;

**Beispiel12**
Kapseln enthalten:
200 mg $\beta$-Hydroxyethyl-rutoside;
300 mg D-alpha-Tocopherolacetat;
180 mg Sojaöl;

**Beispiel13**
Kapseln enthalten:
150 mg Extract Hippocastani (enthalten 25 mg Aescin);
300 mg D-alpha-Tocopherol;
150 mg Sojaöl;

**Beispiel14**
Kapseln enthalten:
300 mg Xantinolnicotinat;
400 mg D-alpha-Tocopherol;
190 mg Sojaöl;

**Beispiel15**
Kapseln enthalten: 150 mg Extract Hippocastani (enthalten 25 mg Aescin);
250 mg Vitamin E;
150 mg Sojaöl;

**Beispiel16**
Kapseln enthalten:
5 mg Vitamin $B_1$;
5 mg Vitamin $B_2$;
5 mg Vitamin $B_6$;

6

200 mg β-Hydroxyethyl-rutoside;
300 mg Vitamin E;
50 mg Nicotinsäureamid;
200 mg Sojaöl;

**Beispiel17**
Kapseln enthalten:
100 mg Nicotinsäure;
100 mg Rosskastanienextract (enthalten 16 mg Aescin);
300 mg D-alpha-Tocopherolacetat;
200 mg Sojaöl;

**Beispiel18**
Kapseln enthalten:
200 mg Inositol Nicotinat;
300 mg D-alpha-Tocopherol-Konzentrat;
150 mg Sojaöl;

**Beispiel19**
Kapseln enthalten:
50 mg Procainhydrochlorid;
400 mg D-alpha-Tocopherol-Konzentrat;
150 mg Sojaöl;

**Beispiel20**
50 mg Procainhydrochlorid;
400 mg D,L-alpha-Tocopherolacetat;
5 mg Vitamin $B_1$;
5 mg Vitamin $B_2$;
5 mg Vitamin $B_6$;
150 mg Sojaöl oder Maisöl;

**Beispiel 21**
Tropfen
100 ml 90% Ethylalkohol enthalten
40 g D,L-alpha-Tocopherolacetat;
4,5 g Extract Hippocastani (enthalten 750 mg Aescin);

**Beispiel 22**
Kapseln enthalten:
4,5 mg entsprechend Dihydroergotoxin-methan-sulfonat;
400 mg D,L-alpha-Tocopherolacetat;

**Beispiel 23**
Kapseln enthalten:
50 mg Procain-Hydrochlorid;
200 mg Nicotinsäure;
400 mg Vitamin E;
150 mg Maisöl.

**Beispiel 24**
Kapseln enthalten:
150 mg Bencyclan-hydrogenfumarat;
400 mg Vitamin E als D,L-alpha-Tocopherolacetat;
150 mg Sojaöl.

**Beispiel25**
Suppositorien enthalten:

450 mg D-alpha-Tocopherol-Konzentrat
30 mg Nicotinsäurebenzylester
100 mg getrockneten, enteiweißten, wässrigen Auszug aus Testis bovis
70 mg Extract Muirae puamae sicc.
ad 2,0 g Stadimol

**Beispiel 26**
Suppositorien enthalten:
450 mg D,L-alpha-Tocopherol
40 mg Cetiol$^{(R)}$ (Ölsäureoleylester)
150 mg Zinkoxid
ad 2,0 g Stadimol

**Beispiel 27**
Suppositorien enthalten:
400 mg Vitamin E
200 mg $\beta$-Hydroxyethylrutoside
40 mg Cetiol$^{(R)}$
ad 2,0 g Stadimol

**Beispiel 28**
Suppositorien enthalten:
350 mg Vitamin E
250 mg Extract Hippocastani (enthält ca. 80 mg Aescin)
ad 2,0 g Stadimol

**Beispiel 29**
Gemäß Beispiel 27 wurden Suppositorien hergestellt, jedoch mit
300 mg Vitamin E und
200 mg Tri-ethylolrutoside

**Beispiel 30**

| Kapseln enthalten: | |
| --- | --- |
| Pentoxyfyllin | 400 mg |
| Vitamin E | 400 mg |
| Vitamin A Acetat | 15.000 I.E. |
| Sojaöl | 120 mg |

**Beispiel 31**

| Kapseln enthalten: | |
| --- | --- |
| Naftidirofuryl-Hydrogenoxalat | 100 mg |
| Vitamin E | 500 mg |
| Vitamin A Palmitat | 30.000 I.E. |
| Sojaöl | 150 mg |

**Beispiel32**

Kapseln enthalten:

| | |
|---|---|
| Cinnarizin | 75 mg |
| Vitamin E | 400 mg |
| Vitamin A Palmitat | 15.000 I.E. |
| Vitamin $B_1$, $B_2$, $B_6$ zu gleichen Teilen | 10 mg |
| Vitamin $B_{12}$ | 5 $\mu$g |
| Sojaöl | 150 mg |

**Beispiel33**

100 ml Tropfen aus Ethylalkohol enthalten:

| | |
|---|---|
| Cinnarizin | 7,5 g |
| Vitamin E | 4,0 g |
| Vitamin A Palmitat | 2,5 Millionen Einheiten |

**Beispiel 34**

Kapseln enthalten:

| | |
|---|---|
| Xantinolnicotinat | 500 mg |
| Vitamine E (DL-alpha-Tocopherol) | 400 mg |
| Vitamin A Palmitat | 10.000 I.E. |
| Tween[R] 80 | 20 mg |
| Sojaöl | 150 mg |

**Beispiel 35**

Tropfen in 100 ml Ethylalkohol:

Dihydroergotoxinmethansulphonat          1,5 g

aus (0,5 g Dihydroergocristinmethansulfonat)

0,5 g Dihydroergocorninmethansulfonat

0,333 mg alpha-Dihydroergocryptinmethansulfonat

0,167 mg ß-Dihydroergocryptinmethansulfonat)

Vitamin E (DL-alpha-Tocopherolacetat) 3,5 g

Vitamin A Palmitat      2,5 Millionen Einheiten


**Beispiel 36**


Kapseln enthalten:

ß-Pyridyl-carbinol-tartrat          360 mg

entspricht 150 mg Pyridylcarbinol

D-alpha-Tocopherolacetat          400 mg

Vitamin A Palmitat          12.000 I.E.



        Sojaöl          150 mg


**Beispiel 37**


Kapseln enthalten:

D,L-alpha-Tocopherol          400 mg

ß-Hydroxyethylrutosid          300 mg

Vitamin A Palmitat          15.000 I.E.

Sojaöl          150 mg


**Beispiel 38**

Kapseln enthalten:

| | |
|---|---|
| Ginkoflavonglykoside | 3,0 mg |
| Vitamin E (D,L-alpha-Tocopherol-acetat) | 300 mg |
| Vitamin A Palmitat | 25.000 I.E. |
| Sojaöl | 100 mg |

**Beispiel** 39

Kapseln enthalten:

| | |
|---|---|
| Nicotinsäure · | 300 mg |
| Vitamin E | 400 mg |
| Vitamin A Palmitat | 15.000 I.E. |
| Cetiol(R) | 20 mg |
| Sojaöl | 150 mg |

**Beispiel** 40

Kapseln enthalten:

| | |
|---|---|
| D,L-alpha-Tocopherolacetat | 200 mg |
| ß-Hydroxyethylrutosid | 300 mg |
| Vitamin A Palmitat | 25.000 I.E. |
| Sojaöl | 120 mg |

**Beispiel** 41

Kapseln enthalten:

| | |
|---|---|
| Pentoxyfyllin | 400 mg |
| Vitamin E (D,L-alpha-Tocopherol-acetat) | 400 mg |
| Vitamin A Palmitat | 15.000 I.E. |
| Tween(R) 80 | 10 mg |
| Sojaöl | 150 mg |

11

**Beispiel** 42

Kapseln enthalten:

| | |
|---|---|
| Bamethansulfat | 25 mg |
| D,L-alpha-Tocopherolacetat | 250 mg |
| Vitamin A Palmitat | 10.000 I.E. |
| Sojaöl | 150 mg |

**Beispiel** 43

Kapseln enthalten:

| | |
|---|---|
| Vincamin | 30 mg |
| Vitamin E (D,L-alpha-Tocopherol-acetat) | 400 mg |
| Vitamin A Palmitat | 30.000 I.E. |
| Sojaöl | 150 mg |

**Beispiel** 44

Eine Salbe enthält:
10 g D-alpha-Tocopherol
50.000 I.E. Heparin Natrium
ad 100 Salbengrundlage aus
22 T Cetyl-Stearylalkohol
18 T Cetiol(R)
60 T Wasser (aqua conservata)

**Beispiel** 45

Eine Salbe enthält:
7 g Vitamin E (D-alpha-Tocopherol)
1 g Nicotinsäurebenzylester
1 g Kampfer
ad 100 Salbengrundlage aus
17 T Cetyl-Stearylalkohol
8 T Weiße Vaseline
15 T Cetiol(R)
60 T Wasser (aqua conservata)

**Beispiel** 46

Eine Salbe enthält:
7 g Vitamin E
15 g Tinct. calendualae
ad 100 Salbengrundlage aus
13 T Wollwachsalkohol
2 T Cetyl-Stearylalkohol
20 T cetiol(R)
5 T Paraffin
50 T Wasser (aqua conservata)

12

**Beispiel47**

Eine Salbe enthält:

8 g Vitamin E (D,L-alpha-Tocopherol)

1,5 g Rosmarinöl

1 g Extract Hippocastani (standardirisiert auf mind. 8% Aescin)

1 g Öl juniperi

ad 100 Salbengrundlage wie Beispiel 44

**Beispiel 48**

Lösung aus

10 g Vitamin E (D-alpha-Tocopherol-Konzentrat)

1 g Latschenkiefernöl (Öl pini pumilionis)

1 g Eucalyptusöl

1 g Öl juniperi

ad 100 Isopropylalkohol

**Beispiel 49**

Eine Salbe enthält:

7 g D-alpha-Tocopherol-Konzentrat

2 g Tinct. arnicae

2 g Salicyl-$\beta$-Hydroxyäthylester

ad 100 Salbengrundlage wie Beispiel 44

**Beispiel 50**

Lösung gemäß Beispiel 48:

7,0 g Vitamin E

1,0 g Latschenkiefernöl

1,0 g Arnikatinktur

ad 100 Isopropylalkohol

**Beispiel 51**

Eine Salbe enthält:

9,0 g Vitamin E

20,0 g Tinct. calendulae

ad 100 Salbengrundlage wie Beispiel 44

Beispiele 52 bis 66 betreffen Kombinationen von Vitamin E und A mit Lecithin.

**Beispiel 52**

| Kapseln enthalten: | |
|---|---|
| Pentoxyfyllin | 400 mg |
| Vitamin E D,L-alpha-Tocopherolacetat | 400 mg |
| Vitamin-A-Acetat | 25.000 I.E. |
| Sojalecithin | 200 mg |
| Sojaöl | 120 mg |
| Tween(R) 80 | 8 mg |

**Beispiel 53**

Kapseln enthalten:

| | |
|---|---|
| Naftidirofuryl-Hydrogenoxalat | 100 mg |
| Vitamin E (D-alpha-Tocopherol-Konzentrat) | 500 mg |
| Vitamin-A-Palmitat | 30.000 I.E. |
| Sojalecithin | 25 mg |
| Sojaöl | 150 mg |

**Beispiel 54**

Kapseln enthalten:

| | |
|---|---|
| Cinnarizin | 75 mg |
| Vitamin E- D-alpha Tocopherolacetat | 400 mg |
| Vitamin-A-Palmitat | 25.000 I.E. |
| Vitamin $B_1$, $B_2$, $B_6$ zu gleichen Teilen | 10 mg |
| Vitamin $B_{12}$ | 5 $\mu$g |
| Sojaöl | 100 mg |
| Sojalecithin | 280 mg |

**Beispiel 55**

100 ml Tropfen aus Ethylalkohol enthalten:

| | |
|---|---|
| Cinnarizin | 7,5 g |
| Vitamin E | 4,0 g |
| Vitamin-A-Palmitat | 2,5 Millionen Einheiten |
| Lecithin | 2,5 g |

**Beispiel 56**

14

Kapseln enthalten:

| | |
|---|---|
| Xantinolnicotinat | 500 mg |
| Vitamin E (D,L-alpha-Tocopherol) | 400 mg |
| Vitamin-A-Palmitat | 25.000 I.E. |
| Tween$^{(R)}$ 80 | 20 mg |
| Sojaöl | 150 mg |
| Sojalecithin | 25 mg |

**Beispiel** 57

Tropfen in 100 ml Ethylalkohol

| | |
|---|---|
| Dihydroergotoxinmethansulfonat | 1,6 g |

aus

(0,5 g Dihydroergocristinmethansulfonat

0,5 g Dihydroergocorninmethansulfonat

333 mg alpha-Dihydroergocryptinmethansulfonat

167 mg ß-Dihydroergocryptinmethansulfonat)

| | |
|---|---|
| Vitamin E (DL-alpha-Tocopherolacetat) | 3,5 g |
| Vitamin-A-Palmitat | 1,5 Millionen Einheiten |
| Sojalecithin | 3,5 g |

**Beispiel** 58

Kapseln enthalten:

| | |
|---|---|
| ß-Pyridyl-carbinol-tartrat | 360 mg |
| entspricht 150 mg Pyridyl-carbinol | |
| D-alpha-Tocopherolacetat | 400 mg |
| Vitamin-A-Palmitat | 10.000 I.E. |
| Sojaöl | 100 mg |
| Sojalecithin | 150 mg |
| Tween$^{(R)}$ 20 | 6 mg |

Beispiel59

Kapseln enthalten:

| | |
|---|---|
| DL-alpha-Tocopherol | 400 mg |
| ß-Hydroxyethylrutosid | 300 mg |
| Vitamin-A-Palmitat | 30.000 I.E. |
| Sojaöl | 100 mg |
| Sojalecithin | 250 mg |

Beispiel 60

Kapseln enthalten:

| | |
|---|---|
| Ginkoflavonglykoside | 3,0 mg |
| Vitamin E DL-alpha-Tocopherol-acetat | 300 mg |
| Vitamin-A-Palmitat | 25.000 I.E. |
| Sojaöl | 100 mg |
| Sojalecithin | 200 mg |

Beispiel61

| | |
|---|---|
| Nicotinsäure | 300 mg |
| Vitamin E | 400 mg |

| | |
|---|---|
| Vitamin-A-Palmitat | 15.000 I.E. |
| Cetiol$^{(R)}$ (Oleylsäureester) | 10 mg |
| Sojaöl | 100 mg |
| Sojalecithin | 20 mg |

Die Produkte der Beispiele 52 bis 61 können als Mittel zur Senkung des Cholesterinspiegels eingesetzt werden.

Die folgenden Beispiele betreffen den Zusatz von Dimethylaminoethanol zu den erfindungsgemäßen Kombinationen.

Beispiel 62

20 mg Dimethylaminoethanol
400 mg D,L-alpha-Tocopherolacetat
12.000 I.E. Vitamin-A-Palmitat (6,67 mg)

50 mg Sojaöl
200 mg Sojalecithin
200 mg β-Hydroxyethylrutosid

**Beispiel** 63
20 mg Dimethylaminoethanol
400 mg D,L-alpha-Tocopherolacetat
12.000 I.E. Vitamin-A-Palmitat (6,67 mg)
100 mg Sojaöl
300 mg Lecithin
75 mg Cinnarizin

**Beispiel** 64
25 mg Dimethylaminoethanolorotat
400 mg D,L-alpha-Tocopherolacetat
15.000 I.E. Vitamin-A-Palmitat (8,33 mg)
20 mg Sojalecithin
400 mg Nicotinsäure

**Beispiel** 65
Wie Beispiel 62, jedoch mit 8 mg Tween$^{(R)}$ 80 angesetzt.

**Beispiel66**
25 mg Dimethylaminoethanolorotat
500 mg D-alpha-Tocopherol-Konzentrat
22.000 I.E. Vitamin-A-Palmitat (12,22 mg)
28 mg Sojalecithin
120 mg Sojaöl
3,0 mg Ginkoflavonglucosid

**Beispiel67**
Kombination gemäß Beispiel 66, jedoch mit 8 mg Tween$^{(R)}$ 20.

**Beispiel68**
30 mg Dimethylaminoethanolorotat
400 mg D,L-alpha-Tocopherolacetat
300 mg Lecithin
8 mg Tween$^{(R)}$ 80
30 mg Vincamin

**Beispiel69**
25 mg Dimethylaminoethanolorotat
350 mg D-alpha-Tocopherolacetat
15.000 I.E. Vitamin-A-Palmitat
je 5 mg von Vitamin $B_1$, $B_2$, $B_6$,
5 μg Vitamin $B_{12}$
15 mg Nicotinsäureamid
280 mg Lecithin
75 mg Cinnarizin

**Beispiel** 70
Kombination gemäß Beispiel 69, jedoch mit 5 mg Tween$^{(R)}$ 80.

**Beispiel** 71
25 mg Dimethylaminoethanolorotat
400 mg D,L-alpha-Tocopherolacetat
15.000 I.E. Vitamin-A-Palmitat (8,33 mg)
300 mg β-Hydroxyethylrutosid

**Beispiel 72**
Kombination gemäß Beispiel 71, jedoch mit 8 mg Tween(R) 80 angesetzt.

**Beispiel 73**
35 mg Dimethylaminoethanolorotat
500 mg D-alpha-Tocopherol-Konzentrat
22.000 I.E Vitamin-A-Palmitat (12,22 mg)
400 mg Xantinolnicotinat

**Beispiel 74**
Gemäß Beispiel 73, jedoch mit 4 mg Tween(R) 20.

**Beispiel 75**
30 mg Dimethylaminoethanolorotat
400 mg D,L-alpha-Tocopherolacetat
400 mg Pentoxyfyllin

**Beispiel 76**
35 mg Dimethylaminoethanolorotat
350 mg D-alpha-Tocopherolacetat
15.000 I.E. Vitamin-A-Palmitat
je 5 mg von Vitamin $B_1$, $B_2$, $B_6$
5 µg Vitamin $B_{12}$
100 mg Bencyclanfumarat

**Beispiel 77**
Kombination gemäß Beispiel 76, jedoch mit 3 mg Tween(R) 80.

**Beispiel 78**
25 mg Dimethylaminoethanolorotat
350 mg D,L-alpha-Tocopherolacetat
17.000 I.E. (9,44 mg) Vitamin-A-Palmitat
70 mg Sojaöl
75 mg Cinnarizin

**Beispiel 79**
20 mg Dimethylaminoethanol
200 mg D,L-alpha-Tocopherolacetat
12.000 I.E. Vitamin-A-Palmitat (6,67 mg)
50 mg Sojaöl
250 mg Sojalecithin

**Beispiel 80**
35 mg Dimethylaminoethanolorotat
400 mg D,L-alpha-Tocopherolacetat
15.000 I.E. Vitamin-A-Palmitat (8,33 mg)
20 mg Sojalecithin

**Beispiel 81**
Kombination gemäß Beispiel 79, jedoch mit 3 mg Tween(R) 80 angesetzt.

**Beispiel 82**
20 mg Dimethylaminoethanol
200 mg D,L-alpha-Tocopherolacetat
12.000 I.E. Vitamin-A-Palmitat (6,67 mg)
50 mg Sojaöl

**Beispiel 83**

35 mg Dimethylaminoethanolorotat
400 mg D,L-alpha-Tocopherolacetat
15.000 I.E. Vitamin-A-Palmitat (8,33 mg)

**Beispiel 84**
Kombination gemäß Beispiel 86, jedoch mit 3 mg Tween[R] 80 angesetzt.

**Beispiel 85**
35 mg Dimethylaminoethanolorotat
500 mg D-alpha-Tocopherol-Konzentrat
22.000 I.E. Vitamin-A-Palmitat (12,22 mg)

**Beispiel 86**
Kombination gemäß Beispiel 85, jedoch mit 4 mg Tween[R] 20.

**Beispiel 87**
30 mg Dimethylaminoethanolorotat
400 mg D,L-alpha-Tocopherolacetat

**Beispiel 88**
35 mg Dimethylaminoethanolorotat
350 mg D-alpha-Tocopherolacetat
15.000 I.E. Vitamin-A-Palmitat
je 5 mg von Vitamin $B_1$, $B_2$, $B_6$
5 $\mu$g Vitamin $B_{12}$
15 mg Nicotinsäureamid

**Beispiel 89**
Kombination gemäß Beispiel 88 jedoch mit 3 mg Tween[R] 80.

**Beispiel 90**
25 mg Dimethylaminoethanolorotat
350 mg D,L-alpha-Tocopherolacetat
17.000 I.E. (9,44 mg) Vitamin-A-Palmitat
70 mg Sojaöl

In allen Beispielen wurde Sojaöl zwischen 50 und 200 mg pro Kapsel zugesetzt. Es können auch andere neutrale Öle wie Olivenöl, Rüböl etc. verwendet werden.

**Patentansprüche**

1. Verwendung von Vitamin E in Kombination mit einem oder mehreren gefäßerweiternden und/oder durchblutungsfördernden Mittel(n) und gegebenenfalls einem oder mehreren Stoffen aus der Gruppe Lecithin, Vitamin A, Vitamine der B-Reihe, Analgetika, Emulgatoren, Lösungsvermittler, Antikoagulantien und übliche Träger- und Hilfsstoffe zur Herstellung eines Arzneimittels gegen rheumatische Erkrankungen.

2. Verwendung nach Anspruch 1, wobei die Arzneimittel in Form äußerlich anwendbarer Darreichungsformen vorliegen.

3. Verwendung nach Anspruch 2, wobei die Arzneimittel in Form von Cremes, Salben, Gelen oder Lotionen vorliegen.

4. Verwendung nach Anspruch 2 oder 3, wobei Vitamin E in Form des freien D-alpha-Tocopherols und/oder D,L-alpha-Tocopherols eingesetzt wird.

5. Verwendung nach einem der Ansprüche 2 bis 4, wobei die Menge an Vitamin E in Form von freiem alpha-Tocopherol im Bereich zwischen 5 und 20 Gew.-%, vorzugsweise im Bereich von 5 bis 10 Gew.-

%, besonders bevorzugt im Bereich von 8 Gew.-%, bezogen auf die Gesamtmenge aller Komponenten, liegt.

6. Verwendung nach einem der Ansprüche 2 bis 5, wobei eine Grundlage eingesetzt wird, die (bezogen auf die Gesamtmenge der Komponenten) 70 bis 30 Gew.-%, vorzugsweise 60 bis 40 Gew.-%, Wasser (aqua conservata), 30 bis 5 Gew.-%, vorzugsweise 25 bis 7 Gew.-%, Cetiol (Oleyloleat), 30 bis 2 Gew.-%, vorzugsweise 25 bis 2 Gew.-%, Cetylstearylalkohol oder andere aliphatische Alkohole und gegebenenfalls Isopropanol als Lösungsvermittler enthält.

7. Verwendung nach einem der Ansprüche 2 bis 6, wobei als gefäßerweiternde und/oder durchblutungsfördernde Mittel Heparin-Natrium, Extract. Hippocastani, Extract. Arnicae, Extract. Capsicae, Ol. Juniperi, Ol. Pini pumilionis, Ol. Eucalypti, Ol. Rosmarinae, Tinct. Camphorae oder Campher, Extract. Calendulae, β-Hydroxyethylrutosid, Trimethylolrutosid, Nicotinsäure oder deren Ester, Benzylnicotinat, Inositolnicotinat, Xantinolnicotinat, Salicylsäure oder deren Ester, β-Hydroxyethylsalicylat, Cinnarizin, Vincamin, Pentoxyfyllin, Bamethansulfat, Buflomedil, Bencyclanfumarat, Bencyclanhydrogenfumarat, Flunarizin, Dihydroergotoxinmethansulfonat, β-Pyridylcarbinol und/oder Ginkoflavonglykoside eingesetzt werden.

8. Verwendung nach Anspruch 2, wobei die Arzneimittel in Form von einem Pflaster vorliegen.

9. Verwendung nach einem der Ansprüche 2 oder 8, wobei die Menge an Vitamin E im Bereich von 0,02 bis 4 g, vorzugsweise von 0,1 bis 3 g, pro Pflaster liegt.

10. Verwendung nach einem der Ansprüche 2, 8 oder 9, wobei als gefäßerweiternde und/oder durchblutungsfördernde Mittel Extract. Arnicae, Extract. Hippocastani und/oder Extract. Capsicae eingesetzt werden.

11. Verwendung nach einem der Ansprüche 2, 8, 9 oder 10, wobei zusätzlich ein schmerzlindernder Stoff, vorzugsweise Extract. Belladonnae, eingesetzt wird.

12. Verwendung nach Anspruch 1, wobei die Arzneimittel in Form oral anwendbarer Darreichungsformen vorliegen.

13. Verwendung nach Anspruch 12, wobei die Arzneimittel in Form von Kapseln, vorzugsweise Weichgelatine-Kapseln, vorliegen.

14. Verwendung nach einem der Ansprüche 12 oder 13, wobei Vitamin E in Form des freien Tocopherols und/oder eines seiner Ester natürlicher oder synthetischer Herkunft eingesetzt wird.

15. Verwendung nach einem der Ansprüche 12 bis 14, wobei die Mengen an Vitamin E im Bereich von 150 bis 600 mg, vorzugsweise im Bereich von 200 bis 600 mg, besonders bevorzugt im Bereich von 300 bis 500 mg, pro Darreichungseinheit liegen.

16. Verwendung nach einem der Ansprüche 12 bis 15, wobei als gefäßerweiternde und/oder durchblutungsfördernde Mittel Extract. Hippocastani, Extract. Arnicae, β-Hydroxyethylrutosid, Trimethylolrutosid, Nicotinsäure oder deren Ester, Benzylnicotinat, Inositolnicotinat, Xantinolnicotinat, Salicylsäure oder deren Ester, β-Hydroxyethylsalicylat, Cinnarizin, Vincamin, Pentoxyfyllin, Bamethansulfat, Buflomedil, Bencyclanfumarat, Bencyclanhydrogenfumarat, Flunarizin, Dihydroergotoxinmethansulfonat, β-Pyridylcarbinol und/oder Ginkoflavonglykoside eingesetzt werden.

17. Verwendung nach Anspruch 1, wobei die Arzneimittel in Form rektal anwendbarer Darreichungsformen, bevorzugt in Form von Suppositorien, vorliegen.

18. Verwendung nach Anspruch 17, wobei als gefäßerweiternde und/oder durchblutungsfördernde Mittel Extract. Hippocastani, β-Hydroxyethylrutosid, Rutosid-Derivate mit mehreren Hydroxyethylgruppen, Nicotinsäure oder deren Ester, Benzylnicotinat und/oder β-Hydroxyethylnicotinat eingesetzt werden.

19. Verwendung nach einem oder mehreren der Ansprüche 1 bis 18, wobei zusätzlich Vitamin A, bevorzugt

20

in Form von Vitamin A-Palmitat, Vitamin A-Acetat und/oder β-Carotin, besonders bevorzugt in einer Menge von bis zu 25000 i. E. pro Darreichungseinheit, eingesetzt wird.

**Claims**

1. Use of vitamin E in combination with one or more vasodilators and/or blood circulation-promoting agent-(s) and optionally one or more substances from the group of lecithin, vitamin A, vitamins of the B-series, analgesics, emulsifiers, solutizers, anticoagulants, and conventional carriers and auxiliary agents for the preparation of a medicament against rheumatic diseases.

2. The use according to claim 1, wherein the medicaments are in the form of externally applicable administration forms.

3. The use according to claim 2, wherein the medicaments are in the form of creams, ointments, gels or lotions.

4. The use according to claims 2 or 3, wherein vitamin E is employed in the forms of free D-alpha-tocopherol and/or D,L-alpha-tocopherol.

5. The use according to anyone of claims 2 to 4, wherein the amount of vitamin E in the form of free D-alpha-tocopherol is within the range of between 5 and 20% by weight, preferably from 5 to 10% and particularly preferred of 8%, relative to the total amount of all components.

6. The use according to anyone of claims 2 to 5, wherein a base composition is employed which contains (relative to the total amount of the components) from 70 to 30% by weight, and preferably from 60 to 40% by weight, of water (aqua conservata), from 30 to 5% by weight, and preferably from 25 to 7% by weight, of Cetiol (oleyl oleate), and from 30 to 2% by weight, and preferably from 25 to 2% by weight, of cetylstearylalcohol or other aliphatic alcohols and optionally isopropanol as solutizer.

7. The use according to anyone of claims 2 to 6, wherein heparin sodium, Extract. hippocastani, Extract. arnicae, Extract. capsicae, Ol. juniperi, Ol. pini pumilionis, Ol. eucalypti, Ol. rosmarinae, Tinct. camphorae or camphor, Extract. calendulae, β-hydroxyethyl rutoside, trimethylol rutoside, nicotinic acid or esters thereof, benzyl nicotinate, inositol nicotinate, xanthinol nicotinate, salicylic acid or esters thereof, β-hydroxyethyl salicylate, Cinnarizine, Vincamine, Pentoxyfylline, Bamethan sulfate, Buflomedil, Bencyclan fumarate, Bencyclan hydrogenfumarate, Flunarizine, dihydroergotoxine methanesulfonate, beta-pyridylcarbinol, and/or Gingko flavoglycosides are used as the vasodilators and/or as the blood circulation-promoting agents.

8. The use according to claim 2, wherein the medicaments are present in the form of a plaster.

9. The use according to anyone of claims 2 to 8, wherein the amount of vitamin E is within the range of from 0.02 to 4 g, and preferably from 0.1 to 3 g, per plaster.

10. The use according to anyone of claims 2, 8 or 9, wherein Extract. arnicae, Extract. hippocastani and/or Extract. capsicae are used as the vasodilators and/or as the blood circulation-promoting agents.

11. The use according to anyone of claims 2, 8, 9 or 10, wherein a pain-alleviating agent, and preferably Extract. Belladonnae, is employed.

12. The use according to claim 1, wherein the medicaments are present in the form of an orally applicable dosage form.

13. The use according to claim 12, wherein the medicaments are present in the form of capsules, and preferably soft gelatin capsules.

14. The use according to anyone of claims 12 or 13, wherein vitamin E is employed in the forms of free D-alpha-tocopherol and/or one of its esters of natural or synthetic origin.

21

15. The use according to anyone of claims 12 to 14, wherein the amounts of vitamin E are within the range of from 150 to 600 mg, preferably within the range of from 200 to 600 mg and particularly preferred within the range of from 300 to 500 mg, per dosage unit.

16. The use according to anyone of claims 12 to 15, wherein Extract. hippocastani, Extract. arnicae, β-hydroxyethyl rutoside, trimethylol rutoside, nicotinic acid or esters thereof, benzyl nicotinate, inositol nicotinate, xanthinol nicotinate, salicylic acid or esters thereof, β-hydroxyethyl salicylate, Cinnarizine, Vincamine, Pentoxyfylline, Bamethan sulfate, Buflomedil, Bencyclan fumarate, Bencyclan hydrogenfumarate, Flunarizine, dihydroergotoxine methanesulfonate, betapyridylcarbinol, and/or Gingko flavoglycosides are used as the vasodilators and/or as the blood circulation-promoting agents.

17. The use according to claim 1, wherein the medicaments are present in the form of a rectally applicable dosage form, and preferably in the form of suppositories.

18. The use according to claim 17, wherein Extract. hippocastani, β-hydroxyethyl rutoside, rutoside derivatives comprising several hydroxyethyl groups, nicotinic acid or esters thereof, benzyl nicotinate and/or β-hydroxyethyl nicotinate are used as the vasodilators and/or as the blood circulation-promoting agents.

19. The use according to one or more of claims 1 to 18, wherein vitamin A is additionally employed, preferably in the forms of vitamin A palmitate, vitamin A acetate and/or β-carotene, and especially preferred in an amount of up to 25,000 I.U. per dosage unit.

**Revendications**

1. Utilisation de la vitamine E combinée à une ou plusieurs substances vasodilatatrices et/ou favorisant la circulation du sang et éventuellement à une ou plusieurs substances du groupe comprenant la lécithine, la vitamine A, des vitamines de la série B, des analgésiques, des émulsifiants, des agents de solubilisation, des anticoagulants et des excipients et adjuvants usuels, pour préparer un medicament contre des maladies rhumatismales.

2. Utilisation selon la revendication 1, caractérisée en ce que les médicaments se présentent sous forme d'unités d'administration à application externe.

3. Utilisation selon la revendication 2, caractérisée en ce que les médicaments se présentent sous forme de crèmes, de pommades, de gels ou de lotions.

4. Utilisation selon la revendication 2 ou 3, caractérisée en ce qu'on utilise la vitamine E sous forme de D-alpha-tocophérol libre et/ou de D,L-alpha-tocophérol.

5. Utilisation selon l'une des revendications 2 à 4, caractérisée en ce que la quantité de vitamine E, sous forme d'alpha-tocophérol libre, est comprise entre 5 et 20% en poids, de préférence entre 5 et 10 % en poids, et d'une manière plus préférentielle est de l'ordre de 8 % en poids, par rapport à la quantité totale de tous les composants.

6. Utilisation selon l'une des revendications 2 à 5, caractérisée en ce qu'on utilise un produit de base qui contient, par rapport à la quantité totale des composants, 70 à 30 % en poids, de préférence 60 à 40 % en poids d'eau (aqua conservata), 30 à 5 % en poids, de préférence 25 à 7% en poids de cétiol (oléate d'oléyle), 30 à 2 % en poids, de préférence 25 à 2 % en poids d'alcool cétylstéarylique ou d'autres alcools aliphatiques, et éventuellement de l'isopropanol comme agent de solubilisation.

7. Utilisation selon l'une des revendications 2 à 6, caractérisée en ce qu'on utilise comme substances vasodilatatrices et/ou favorisant la circulation du sang, l'héparine sodique, l'extract. hippocastani, l'extract. arnicae, l'extract. capsicae, l'ol. juniperi, l'ol. pini pumilionis, l'ol. eucalypti, l'ol. rosmarinae, la tinct. camphorae ou le camphre, l'extract. calendulae, le β-hydroxyéthylrutoside, le triméthylolrutoside, l'acide nicotinique ou ses esters, le nicotinate de benzyle, le nicotinate d'inositol, le nicotinate de xantinol, l'acide salicylique ou ses esters, le salicylate de β-hydroxyéthyle, la cinnarizine, la vincamine, la pentoxyfylline, le sulfate de baméthan, le buflomédil, le fumarate de bencyclane, l'hydrogénofumarate de bencyclane, la flunarizine, le méthanesulfonate de dihydroergotoxine, le $\beta$-pyridylcarbinol et/ou des glucosides de ginkgoflavone.

8. Utilisation selon la revendication 2, caractérisée en ce que les médicaments se présentent sous forme d'emplâtre.

9. Utilisation selon l'une des revendications 2 et 8, caractérisée en ce que la quantité de vitamine E est comprise entre 0,02 et 4 g, de préférence entre 0,1 et 3 g, par emplâtre.

10. Utilisation selon l'une des revendications 2, 8 et 9, caractérisée en ce qu'on utilise comme substances vasodilatatrices et/ou favorisant la circulation du sang, l'extract. arnicae, l'extract. hippocastani et/ou l'extract. capsicae.

11. Utilisation selon l'une des revendications 2, 8, 9 et 10, caractérisée en ce qu'on utilise en outre une substance analgésique, de préférence l'extract. belladonae.

12. Utilisation selon la revendication 1, caractérisée en ce que les médicaments se présentent sous forme d'unités d'administration à application par voie buccale.

13. Utilisation selon la revendication 12, caractérisée en ce que les médicaments se présentent sous forme de capsules, de préférence des capsules de gélatine molle.

14. Utilisation selon l'une des revendications 12 et 13, caractérisée en ce qu'on ajoute la vitamine E sous forme de tocophérol libre et/ou d'un de ses esters d'origine naturelle ou synthétique.

15. Utilisation selon l'une des revendications 12 à 14, caractérisée en ce que les quantités de vitamine E sont comprises entre 150 et 600 mg, de préférence entre 200 et 600 mg, et d'une manière plus préférentielle, entre 300 et 500 mg, par unité d'administration.

16. Utilisation selon l'une des revendications 12 à 15, caractérisée en ce qu'on utilise comme substances vasodilatatrices et/ou favorisant la circulation du sang, l'extract. hippocastani, l'extract. arnicae, le $\beta$-hydroxyéthylrutoside, le triméthylolrutoside, l'acide nicotinique ou ses esters, le nicotinate de benzyle, le nicotinate d'inositol, le nicotinate de xantinol, l'acide salicylique ou ses esters, le salicylate de $\beta$-hydroxyéthyle, la cinnarizine, la vincamine, la pentoxyfylline, le sulfate de baméthan, le buflomédil, le fumarate de bencyclane, l'hydrogénofumarate de bencyclane, la flunarizine, le méthanesulfonate de dihydroergotoxine, le $\beta$-pyridylcarbinol et/ou des glucosides de gingkoflavone.

17. Utilisation selon la revendication 1, caractérisée en ce que les médicaments se présentent sous forme d'unités d'administration à application par voie rectale, de préférence sous forme de suppositoires.

18. Utilisation selon la revendication 17, caractérisée en ce qu'on utilise comme substances vasodilatatrices et/ou favorisant la circulation du sang, l'extract. hippocastani, le $\beta$-hydroxyéthylrutoside, des dérivés de rutoside avec plusieurs groupes hydroxyéthyle, l'acide nicotinique ou ses esters, le nicotinate de benzyle et/ou le nicotinate de $\beta$-hydroxyéthyle.

19. Utilisation selon l'une ou plusieurs des revendications 1 à 18, caractérisée en ce qu'on utilise en outre de la vitamine A, de préférence sous forme de vitamine A-palmitate, de vitamine A-acétate et/ou de $\beta$-carotine, d'une manière plus préférentielle, en une quantité allant jusqu'à 25.000 U.I. par unité d'administration.

23